# EUROPEAN PATENT APPLICATION

(11) **EP 3 805 689 A1**
(43) Date of publication of application: **14.04.2021**
(21) Application number: 19201662.4
(22) Date of filing: 07.10.2019
(51) Int. Cl.: F28G 13/00, F28D 9/00, A61L 2/03

(54) **PREVENTION OF MICROBIOLOGICAL GROWTH IN HEAT EXCHANGERS**

(71) Applicant: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(57) **Abstract**

A heat exchanger assembly (10) is proposed comprising: a heat exchanger (12) forming one or more walls (26) separating a first fluid (22) and a second fluid (24). The assembly (10) further comprises: a first electrical connector (14) and a second electrical connector (16) that are operationally connected to the walls (26) of the heat exchanger (12) and an electrical power source (18) operationally connected to the first electrical connector (14) and the second electrical connector (16). The electrical power source (18) is configured to supply an electric current to the one or more walls (26) of the heat exchanger (12) via the first electrical connector (14) and the second electrical connector (16) .

## Description

### Technical field

The proposed technology relates generally to the field of heat exchangers. It relates specifically to plate heat exchangers and heat exchangers in marine applications. The technology also relates to the prevention of the growth of microorganisms on the inside surfaces of heat exchangers.

### Background

Heat exchangers are systems that are used to transfer heat between two or more fluids. Typically, the fluids are separated by a solid wall to prevent mixing. Heat exchangers are used in both cooling and heating processes.

Microbiological growth in heat exchangers is a recognized problem. Such growth can negatively affect the performance of the heat exchanger, for example with respect to flow rates and heat conductance. Microbiological growth is particularly a problem in marine applications, in which sea water is supplied to the heat exchangers. Sea water is high in nutrients and the temperature of the supplied sea water is often within a range that is favorable for microbiological growth.

### Object

The proposed technology aims at preventing or reducing microbiological growth in heat exchangers, and in particular on the walls of heat exchangers.

### Summary

In a first aspect of the proposed technology, a heat exchanger assembly is provided. The assembly comprises: a heat exchanger forming one or more walls separating a first fluid and a second fluid and through which heat can be transferred between the first fluid and the second fluid. The assembly further comprises: a first electrical connector and a second electrical connector that are operationally connected to the one or more walls of the heat exchanger. Additionally, the assembly comprises an electrical power source operationally connected to the first electrical connector and the second electrical connector and configured to supply an electric current, and/or an electric potential, to the one or more walls of the heat exchanger via the first electrical connector and the second electrical connector.

It is understood that the first aspect of the proposed technology is directed to a heat exchanger assembly for reducing, inhibiting, or preventing growth of microorganisms in the heat exchanger forming part of the assembly. Operationally connected is here understood to only specify that there is an electrical connection by which a current can be supplied.

The first fluid and the second fluid may be liquids. The assembly allows for the prevention of growth of microorganisms on the one or more walls. The electrical power source may be configured to supply an electric current or electric potential for reducing, inhibiting, or preventing growth of microorganisms in the heat exchanger, or on the one or more walls of the heat exchanger. In extension, the assembly allows for a reduction of the generation or growth of a biofilm on the one or more walls. Microorganisms are here understood to encompass unicellular organisms that may exist in single-celled forms or in colonies of cells.

The growth conditions may depend on microbial nutrients, temperature, and oxygen level in the fluids. For example, sea water at about 40 degrees C provides better growth condition than chlorinated tap water at about 10 degrees C.

It is understood that the one or more walls are electrically conductive. They may be formed of a metal or a combination of metals, e.g. an alloy. It is also understood that one of the electrical connectors, such as the second electrical connector, may be grounded or earthed, for example by way of the hull of a ship. If an alternating electric current is supplied by the electrical power source, this means that the neutral and earth are shared and that the phase is provided by the other electrical connector.

It is understood that the heat exchanger may be a shell and tube heat exchanger, plate heat exchanger, plate and shell heat exchanger, plate fin heat exchanger, or a pillow plate heat exchanger.

The first electrical connector and the second electrical connector may be spaced apart at the heat exchanger.

The one or more walls may further contain or enclose the first fluid and the second fluid from inside the heat exchanger. This means that both the first fluid and the second fluid are surrounded by the one or more walls when they pass through the heat exchanger, and that the flow of the first fluid and the second fluid is restricted by the one or more walls when passing through the heat exchanger. The one or more walls may form a plurality of walls.

In a second aspect of the proposed technology, a method is provided for reducing, inhibiting, or preventing microbiological growth in a heat exchanger of a heat exchanger assembly according to the first aspect of the proposed technology. The method comprises: supplying an electric current, and/or an electric potential, to the one or more walls of the heat exchanger with the electrical power source. The method may further comprise: providing a flow of the first fluid and a flow of the second fluid in the heat exchanger. The electric current, and/or an electric potential, may be configured for reducing, inhibiting, or preventing growth of microorganisms in the heat exchanger, or on the one or more walls of the heat exchanger.

In a third aspect of the proposed technology, a system is provided for preventing microbiological growth in a heat exchanger forming one or more walls separating a first fluid and a second fluid and through which heat can be transferred between the first fluid and the second fluid. The system comprises: a first electrical connector and a second electrical connector adapted for operationally connecting to the one or more walls of the heat exchanger. Additionally, the assembly comprises: an electrical power source adapted for operationally connecting to the first electrical connector and the second electrical connector and configured to supply an electric current, and/or an electric potential, to the one or more walls of the heat exchanger via the first electrical connector and the second electrical connector.

As for the first aspect of the proposed technology, the electrical power source may be configured to supply an electric current or electric potential for reducing, inhibiting, or preventing growth of microorganisms in the heat exchanger, or on the one or more walls of the heat exchanger.

In a fourth aspect of the proposed technology, a method is provided for reducing, inhibiting, or preventing microbiological growth in a heat exchanger forming one or more walls separating a first fluid and a second fluid and through which heat can be transferred between the first fluid and the second fluid. The method comprises: providing a system according to the third aspect of the proposed technology, operationally connecting the first electrical connector and the second electrical connector to the one or more walls of the heat exchanger, and supplying an electric current, and/or an electric potential, to the one or more walls of the heat exchanger with the electrical power source. The method may further comprise: providing a flow of the first fluid and a flow of the second fluid in the heat exchanger. The electric current, and/or an electric potential, may be configured for reducing, inhibiting, or preventing growth of microorganisms in the heat exchanger, or on the one or more walls of the heat exchanger.

Optional features of the proposed technology are described below.

The heat exchanger may have more than one wall and the walls may be electrically connected or coupled to one another. This means that the walls are not electrically insulated from one another, for example by non-conductive spacers separating the walls. The walls being electrically connected may be achieved by the walls contacting each other, for example by pressing the walls together. It may also be achieved by the walls being joined, for example by soldering, welding, or brazing, or by the walls being connected by an electrically conductive connector or support. This means that all walls more or less are on the same electric potential.

Apart from the first electrical connector and a second electrical connector operationally connecting to the one or more walls of the heat exchanger, the heat exchanger may be electrically insulated from the surroundings. For example, this may be achieved by the heat exchanger being supported by an electrically insulating support, for example an electrically non-conductive plastic support.

The heat exchanger is configured to handle sea water. The first fluid may be sea water. This means that the first fluid contains salts and typically also significant amounts of nutrients for microorganisms.

The heat exchanger may be a plate heat exchanger comprising a plurality of plates, or parallel plates, forming the one or more walls. For example, the heat exchanger may be composed of 20 plates, of which 2 are outer plates and 18 are inner plates forming 18 walls separating the first fluid and the second fluid. It is understood that the plurality of plates are of an electrically conductive material. The plates may be formed by a metal or a combination of metals, e.g. an alloy. For example, the plates may be formed of stainless steel or titanium.

The plurality of plates may be electrically connected or coupled to one another. For example, this may be achieved by the plates being structurally connected, for example by being pressed together by metal clamps or screws, or by neighboring plates being joined together by welding or brazing. This means that the plates are not electrically insulated from one another, for example by non-conductive spacers separating the plates.

The first electrical connector and the second electrical connector may be structurally connected to different plates of the plurality of plates. This has the effect that the electrical power source can generate an electric potential that on average has a component that is transverse to the plates. It is contemplated that this contributes to an improved performance. Structurally connected to a plate is here understood to encompass the electrical connector being connected directly, or forming physical connection, to the plate. Alternatively, the first electrical connector and the second electrical connector may be structurally connected to the same plates of the plurality of plates.

It is understood that the electrical connectors can be structurally connected to two, or more than two plates, at the same time.

The conditions for microbial growth may be greater in the first fluid than in the second fluid, and the first electrical connector may be structurally connected to a pair of adjacent, or juxtaposed, plates of the plurality of plates, and the pair of adjacent plates may enclose the first fluid. Similarly, the second electrical connector may be structurally connected to the pair of adjacent plates. This contributes to an optimized travel through the plate heat exchanger of the current, preventing microbiological growth.

The first electrical connector and the second electrical connector may be structurally connected to the heat exchanger, or to the plurality of plates, on opposite sides of the heat exchanger. This has the effect that the electrical power source can generate an electric potential having a component that is parallel to and crosses the plates. Structurally connected to the heat exchanger is here understood to encompass the electrical connector being connected directly, or forming physical connection, to the heat exchanger. It is contemplated that this contributes to an optimized travel of the current for preventing microbiological growth.

The plates of the heat exchanger may have the same outer shape, or similar outer shapes, and the plates may be oriented in the same direction.

A plate, or each plate, of the plurality of parallel plates may have four side sections and four corner sections, each corner section being located between two side sections, wherein the first electrical connector is structurally connected to a first corner section, and the second electrical connector is structurally connected to a second corner section, wherein the second corner section is diagonal to the first corner section. The second corner section being diagonal to the first corner section means that said corner sections are connected by a side section, a corner section, and an additional side section on either side of the first corner section.

In a different wording, a plate, or each plate, of the plurality of parallel plates may have a generally rectangular shape with four corners, and the first electrical connector and the second electrical connector may be structurally connected at different corners that are located diagonally with respect to one another, or with respect to the plates.

The electric current may be an alternating current. The alternating current may have a square wave form. This means that the peak voltage is approximately equal to the root-mean square voltage. It has been found that this type of current inhibits microbiological growth.

The alternating current may be below 10 mA, below 1 mA, between 0.1 mA and 1 mA, or between 0.3 mA and 0.7 mA. It has been found that this current is sufficient for inhibiting microbiological growth. The alternating current may have a frequency below 100 Hz, below 10 Hz, or below 1 Hz. The alternating current may be supplied at a peak voltage below 120 V, in the range 40 V to 100 V, or in the range 70 to 90 V. Additionally or alternatively, the alternating current may have a duty cycle of about 50 %.

### Brief description of the drawings

A more complete understanding of the abovementioned and other features and advantages of the proposed technology will be apparent from the following detailed description of preferred embodiments in conjunction with the appended drawings, wherein:
- Fig. 1: is a schematic view of an embodiment of a heat exchanger assembly,
- Fig. 2: is a schematic view of a test setup, and
- Fig. 3: is a graph showing the pressure drop (ΔP) over the heat exchanger in two different test runs.

### Description of the drawings

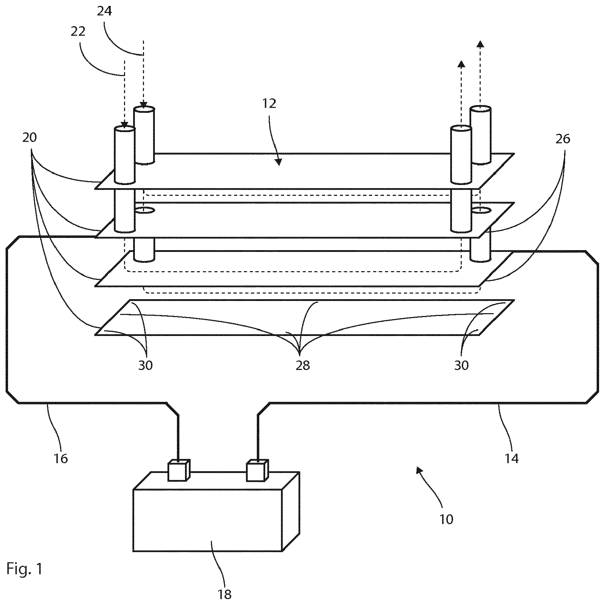

An embodiment of a heat exchanger assembly 10 is schematically illustrated in Fig. 1. It is composed of a heat exchanger 12, a first electrical connector 14, a second electrical connector 16, and an electrical power source 18. The heat exchanger 14 is a plate heat exchanger having four parallel plates 20. The plates are rectangular and of stainless steel. This means that each plate 20 has four side sections 28 and four corner sections 3 as indicated in Fig. 1.

The plates 20 are joined at the edges (not shown) and forms three channels in which a first fluid 22 and a second fluid 24 flow as indicated by the dashed lines in Fig. 1. The first fluid 22 is sea water and the second fluid 24 is fresh water. The flows of the first fluid 22 and the second fluid 24 are generated, or provided by pumps (not shown).

The two central plates 20 form two walls 26 between which the first fluid 22 flows, thus separating the first fluid 22 from the second fluid 24. Heat can be transferred between the first fluid 22 and the second fluid 24 through the two walls 26. In this embodiment, the first fluid 22 has a higher temperature than the second fluid 24 and heat is transferred from the former to the latter.

The first electrical connector 14 and the second electrical connector 16 are electrical wires that are attached to the inner plates 20 by clamps (not shown). The first electrical connector 14 is connected to the lower inner plates 20 and the second electrical connector is connected to the other upper inner plate 20, as is shown in Fig. 1. This means that the first electrical connector 14 and a second electrical connector 16 are structurally connected to different inner plates 20, and also operationally connected to the separating walls 26. They are also attached at diagonally opposite corner sections 30 of the inner plates 20, as is shown in Fig. 1. This means that the first electrical connector 14 and the second electrical connector 16 are spaced apart at the heat exchanger 12, that they are structurally connected to the heat exchanger 12 on opposite sides of the heat exchanger 12, and that they are structurally connected at different corners that are located diagonally with respect to one another.

In an alternative embodiment, the first electrical connector 14 is connected to both inner plates 20 and the second connector 16 is also connected only to both inner plates 20.

The first electrical connector 14 and the second electrical connector 16 are connected to the output terminals of the electrical power source 18. This way the electrical power source 18 can supply an electric current and an electric potential to the separating walls 26 via the first electrical connector 14 and the second electrical connector 16. In an alternative embodiment, the second electrical connector 16 is electrically grounded to earth.

There are rubber gaskets between the plates 20 preventing leakage of the first fluid 22 and the second fluid 24 from the heat exchanger 12. The plates 20 are pressed together and held in place by metal screw clamps (not shown) contacting all plates 20. This means that the plates are electrically connected, and in extension that the separating walls 26 are electrically connected to one another.

The electrical power source 18 supplies an alternating electric current in the form of a square wave to the first electrical connector 14 and the second electrical connector 16, and in extension to the one or more separating walls 26. The alternating current has an electric peak current between 0.3 mA and 0.7 mA, a frequency below 1 Hz, and a duty cycle of about 50 %. The alternating current is supplied at a peak voltage in the range 70 to 90 V.

### Example

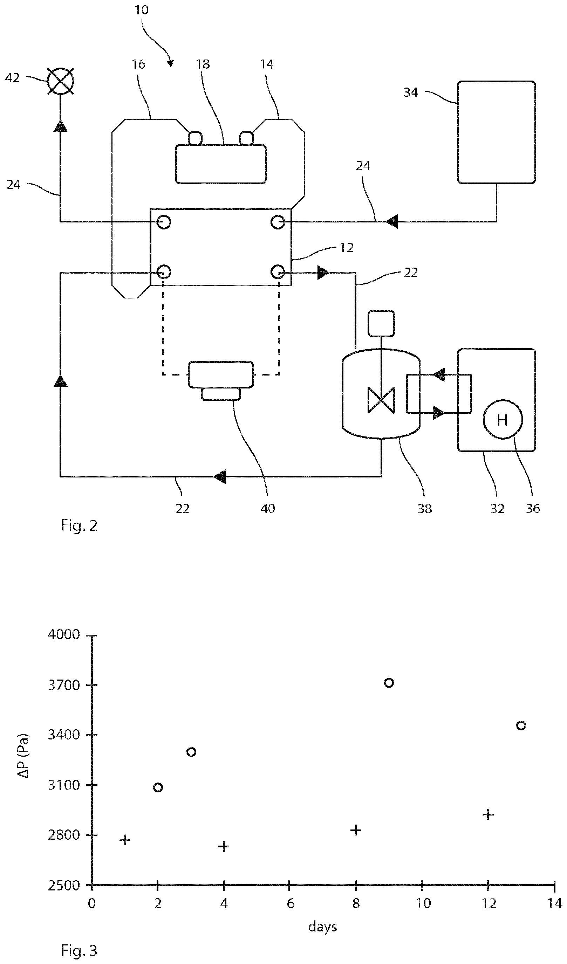

A test setup used in a proof-of-concept is illustrated in Fig. 2. The setup included a heat exchanger assembly 10 as described in relation to Fig.1. The electric conductivity of the stainless steel plates 20 of the heat exchanger 12 is high. Therefore, a 51 kOhm resistor (not shown) was placed in series with the heat exchanger 12 to limit the current.

The test setup has a first tank 32 containing water and a heater 36 arranged to heat the water in the first tank 32. The setup further has a jacketed tank 38 containing brackish sea water that constitutes a first fluid 22. The jacketed tank 38 is coupled to the first tank 32 so that heat can be received therefrom. The test setup also has a second tank 34 containing colder tap water constituting a second fluid 24.

The test setup further has pressure gauge 40. The different components are connected as indicated in Fig. 2. The setup has a number of valves and pumps (not shown) that generates and controls the flows indicated by arrows in Fig. 2. The setup is coupled to a drain 42 such that the tap water in the second tank 34 can empty from the setup after passing the heat exchanger 12. The setup also has a number of thermometers (not shown) for measuring the temperature of the first fluid 22 and the second fluid 24 at the respective inlet of the heat exchanger 12.

The following temperatures were measured by the thermometers: the inlet temperature of the first fluid 22 (Th,in) and the inlet temperature of the second fluid 24 (Tc, in). The pressure drop (ΔP) of the first fluid 22 over the heat exchanger 12 was measured using the pressure gauge 40. The pressure drop (ΔP) of the first fluid 22 was used to characterize the performance of the heat exchanger 12.

Two tests were run on the same setup, one with electrification and one without electrification, i.e. with or without an electric current supplied to the heat exchanger 12 by the electrical power source 18. All other test parameters were the same. The setup was cleaned before each test run. The same first fluid 22 (sea water) was used in both test runs. Both tests were run for 18 days.

The input temperature (Th,in) of the first fluid 22 (hot sea water) was held approximately constant at 40 degrees C, and the input temperature (Tc,in) of the second fluid 24 (cold tap water) was held approximately constant at 10 degrees C. The electrical power source 18 was operated at a peak current of about 0.54 mA, a peak voltage of about 80 V, and a frequency of 5 Hz.

After each test run, the heat exchanger 12 was dismantled and biofilm samples were taken from the inner surfaces of the separating walls 26 of the heat exchanger 12 facing the first fluid 22 (sea water). Sterile cotton swabs were used and the same swabbing pattern was repeated. Approximately 2 cm² were swabbed each time.

For both test runs, four biofilm samples were taken at four different points on the inner surfaces of the separating walls 26. Each sample was then turned into triplicates in order to avoid contamination. The samples were analysed using laser based flow cytometry for cell counting and differentiation of dead and viable bacteria. The average bacterial count is presented in Table 1.

**Table 1: Average bacterial count from samples**

| | Not electrified | Electrified |
|---|---|---|
| Total number bacteria | 3608 | 321 |
| Viable number bacteria | 2264 | 50 |

The electrification of the heat exchanger 12 clearly results in a drop in the count of bacteria, both in total number and in the number of viable bacteria. The total number of bacteria is reduced to about 9 %. The effect is even greater for viable bacteria, for which the number of bacteria is reduced to less than 3 %. It can be concluded that the electrification greatly reduces the bacterial growth on the walls of the heat exchanger facing the first fluid 22 (sea water).

Fig. 3 is a graph showing the pressure drop (ΔP) of the first fluid 22 over the heat exchanger 12 as a function of time. Crosses indicate the results for the electrified setup run, and circles indicate the results for the non-electrified setup run. It can be seen in the graph of Fig. 3 that the pressure drop (ΔP) increases with time for the non-electrified test run, while it is more or less constant for the electrified test run. After 14 days, the pressure difference is about 25% higher without electric current. It is contemplated that this change is caused by the greater microbiological growth in the non-electrified test run than in the electrified test run, and that the growth restricts the flow of the first fluid 22 through the heat exchanger 12. This results in a reduced dynamic pressure after the heat exchanger, which in extension leads to a greater pressure drop (ΔP). It can be concluded that the supply of electric current to the heat exchanger 12 prevents a pressure drop (ΔP) over the heat exchanger that is likely caused by the growth of microorganisms.

### Item list

- 10: heat exchanger assembly
- 12: heat exchanger
- 14: first electrical connector
- 16: second electrical connector
- 18: electrical power source
- 20: plates
- 22: first fluid
- 24: second fluid
- 26: separating walls
- 28: side section
- 30: corner section
- 32: first tank
- 34: second tank
- 36: heater
- 38: jacketed tank
- 40: pressure gauge
- 42: drain

## Claims

1. A heat exchanger assembly (10) comprising:
- a heat exchanger (12) forming one or more walls (26) separating a first fluid (22) and a second fluid (24) and through which heat can be transferred between the first fluid (22) and the second fluid (24),
- a first electrical connector (14) and a second electrical connector (16) that are operationally connected to the one or more walls (26) of the heat exchanger (12), and
- an electrical power source (18) operationally connected to the first electrical connector (14) and the second electrical connector (16) and configured to supply an electric current to the one or more walls (26) of the heat exchanger (12) via the first electrical connector (14) and the second electrical connector (16) for reducing growth of microorganisms in the heat exchanger.

2. The heat exchanger assembly (10) according to claim 1, wherein the heat exchanger (12) has more than one wall and the walls (26) are electrically connected or coupled to one another.

3. The heat exchanger assembly (10) according to claim 1 or 2, wherein the heat exchanger (12) is configured to handle seawater.

4. The heat exchanger assembly (10) according to any of the claims 1 to 3, wherein the heat exchanger (12) is a plate heat exchanger (12) comprising a plurality of plates (20) forming the one or more walls (26).

5. The heat exchanger assembly (10) according to claim 4, wherein the first electrical connector (14) and the second electrical connector (16) are structurally connected to different plates (20) of the plurality of plates (20).

6. The heat exchanger assembly (10) according to claim 4 or 5, wherein the conditions for microbial growth is greater in the first fluid (22) than in the second fluid (24), and the first electrical connector (14) may be structurally connected to a pair of adjacent plates (20) of the plurality of plates (20), wherein the pair of adjacent plates (20) encloses the first fluid (22).

7. The heat exchanger assembly (10) according to any of the claims 4 to 6, wherein the first electrical connector (14) and the second electrical connector (16) are structurally connected to the heat exchanger (12) on opposite sides of the heat exchanger (12).

8. The heat exchanger assembly (10) according to any of the claims 4 to 7, wherein a plate of the plurality of plates (20) has four side sections (28) and four corner sections (30), each corner section (30) being located between two side sections (28), wherein the first electrical connector (14) is structurally connected to a first corner section (30), and the second electrical connector (16) is structurally connected to a second corner section (30), wherein the second corner section (30) is diagonal to the first corner section (30) .

9. The heat exchanger assembly (10) according to any of the claims 4 to 8, wherein a plate of the plurality of plates (20) has a generally rectangular shape with four corners, the first electrical connector (14) and the second electrical connector (16) are structurally connected at different corners that are located diagonally with respect to one another.

10. The heat exchanger assembly (10) according to any of the claims 1 to 9, wherein the electric current is an alternating current.

11. The heat exchanger assembly (10) according to claim 10, wherein the alternating current has a square wave form.

12. The heat exchanger assembly (10) according to claim 10 or 11, wherein the alternating current has an electric peak current, which is below 10 mA, below 1 mA, between 0.1 mA and 1 mA, or between 0.3 mA and 0.7 mA.

13. The heat exchanger assembly (10) according to any of the claims 10 to 12, wherein the alternating current has a frequency below 100 Hz, below 10 Hz, or below 1 Hz.

14. The heat exchanger assembly (10) according to any of the claims 10 to 13, wherein the alternating current is supplied at a peak voltage of below 120 V, in the range 40 V to 100 V, or in the range 70 to 90 V.

15. A method for reducing microbiological growth in heat exchanger (12) of a heat exchanger assembly (10) according to any of the claims 1-14, wherein the method comprises:
providing a flow of the first fluid (22) and a flow of the second fluid (24) in the heat exchanger (12), and supplying an electric current to the one or more walls (26) of the heat exchanger (12) with the electrical power source (18).
